Europäisches Patentamt

European Patent Office

Office européen des brevets

(11) Publication number: **0 279 614**
**A2**

## (12) EUROPEAN PATENT APPLICATION

(21) Application number: **88301236.1**

(22) Date of filing: **15.02.88**

(51) Int. Cl.⁴: **C 12 Q 1/28**

(30) Priority: **17.02.87 US 14864**

(43) Date of publication of application:
**24.08.88 Bulletin 88/34**

(84) Designated Contracting States: **DE GB**

(71) Applicant: **SYNBIOTICS CORPORATION**
**11011 Via Frontera**
**San Diego California 92127 (US)**

(72) Inventor: **Jones, Ruth M.**
**3321 Elliott Street**
**San Diego California 92106 (US)**

(74) Representative: **Harrison, David Christopher et al**
**MEWBURN ELLIS & CO 2/3 Cursitor Street**
**London EC4A 1BQ (GB)**

(54) Stable peroxide substrate formulation.

(57) The present invention provides a storage-stable single bottle enzyme substrate solution for a peroxidase enzyme. The substrate solution comprises an acetate/citrate buffer, soluble tetramethylbenzidine, a stable source of hydrogen peroxide and methanol. The substrate solution only produces a coloured solution in the present of peroxidase.

**0 279 614**

**Description**

## STABLE PEROXIDE SUBSTRATE FORMULATION

The present invention relates to enzyme substrates and in particular to a peroxidase enzyme substrate. Formulation of a substrate for peroxidase generally involves a two bottle system. The first bottle contains a buffered hydrogen peroxide. The second contains tetramethylbenzidine in an appropriate solvent. There are two problems which are usually encountered when the substrate system is formulated in a single bottle. The first is the insolubility of tetramethylbenzidine. The second is that a reaction occurs causing the solution to become colored in the absence of peroxidase enzyme which interf eres with the subsequent enzyme determination. A storage-stable single bottle substrate for peroxidase is desirable.

SUMMARY OF THE INVENTION
A storage-stable, single container enzyme substrate solution for a peroxidase enzyme is provided. The substrate solution comprises soluble tetramethylbenzidine (TMB) and a peroxide source and does not produce a colored solution in the absence of peroxidase. The substrate solution comprises in a single container an acetate/citrate buffer, TMB, a stable source of hydrogen peroxide and methanol.

DESCRIPTION OF THE SPECIFIC EMBODIMENTS
A storage-stable single container enzyme substrate solution for a peroxidase enzyme is provided. The storage-stable substrate solution comprises dissolved tetramethylbenzidine and a hydrogen peroxide source. More particularly, the substrate solution comprises in a single container: an acetate/citrate buffer, dissolved tetramethylbenzidine (TMB), a stable source of peroxide, and methanol. A colored solution does not occur under conventional storage.

The aqueous acetate/citrate buffer has a mole ratio of acetate to citrate of from about 35:1 to about 40:1, an aceta te concentration of about 0.01 to about 1.0 N, to provide a pH of about 5.2 to 6.0. Usually the acetate concentration of the substrate solution is from about 0.1 to about 0.2 N, the citrate concentration is from about 0.0002 to about 0.04 N more particularly about 0.0025 N to about 0.006 N, and the pH is from about 5.4 to about 5.8. Sodium is the normal counterion. The total molarity contributed by the buffer to the substrate solution will be about 0.01 to 0.24, more usually 0.05 to 0.15, preferably about 0.1 M.

A pH value lower than about 5.2 may lead to instability of the TMB and the solution turning blue. A pH significantly higher than 6.0 reduces peroxidase activity and thus the signal is compromised. While the concentration of the buffer is not critical, variation from the limits given for the buffer components tends to degrade performance.

3, 3′, 5, 5′-Tetramethylbenzidine (TMB) is normally present in the substrate solution at a concentration of at least about 0.04 mM and up to saturation. Usually TMB will be present at a concentration of about 0.2 to about 0.8 mM, more usually, at a concentration of about 0.4 mM.

Methanol is normally present at a concentration of about 10 to about 50%, usually 30 to 50%, most usually about 40% v/v of the substrate solution. Higher concentrations reduce the signal by affecting enzyme activity. Lower concentrations do not maintain the desired level of TMB in solution. Methanol is preferred as an auxiliary organic solvent, although other low molecular weight, water soluble oxy organic solvents may find use.

A stable source of peroxide at a concentration sufficient to serve as a peroxidase substrate is also present in the substrate solution. A stable source of peroxide is exemplified by hydrogen peroxide addition complexes such as urea hydrogen peroxide. Urea hydrogen peroxide is usually present at a concentration of about 0.001 M to 0.1, usually from about 0.002 to 0.005 M.

Ancillary additives may also be present. A bacterial growth inhibitor may be included in the substrate formulation. A useful bacterial growth inhibitor is sodium benzoate. The ancillary additives will generally be present in less than about 0.5 weight percent.

The storage-stable aqueous substrate solution of this invention solves the problems formerly encountered with single bottle substrate formulations. TMB remains soluble and retains its activity and the solution does not color under normal storage conditions for extended periods of time.

. The subject substrate solution finds use with peroxidase enzymes, particularly horseradish peroxidase, in a variety of applications. Because of the stability of the enzyme, the absence of the enzyme in physiological media, and the ease of detection, horseradish peroxidase finds wide applications as a label in a variety of diagnostic assays. Illustrative of such assays are ELISA (U.S. Patent No. 3,791,932), Tandem (U.S. Patent No. 4,376,110), etc. Kits can be provided for performing the assays, where the kits would include the subject substrate solution, a conjugate of a receptor usually an antibody or a ligand, bound to the horseradish peroxidase, and such other reagents which may be useful in the assay. (See U.S. Patent No. 3,817,837 for the definitions of ligand and receptor, which definitions are incorporated herein by reference.)

The following example is offered by way of illustration and not by way of limitation.

EXPERIMENTAL
The following formulation was prepared as a single bottle substrate for horseradish peroxidase.

2

Formulation:
Per liter:
13.5 g sodium acetate, trihydrate     (0.1 mol)
0.055 g citric acid, monohydrate     (0.0026 mol)
0.27 g urea hydrogen peroxide     (0.003 mol)
100 mg 3,3′,5,5′-tetramethylbenzidine     (0.4mM)
400 ml methanol     (40%)
1 g sodium benzoate     (0.1%)
$H_2O$     q.i.d.
Final pH 5.6-5.7

The above formulation was stable for 1 week at 37°C as evidenced by the absence of discernible color and the solution's usefulness in an assay with horseradish peroxidase (HRP). Specifically, TMB was measured at $OD_{285}$ at a 1:10 dilution. On day 0 TMB measured 0.95. After incubation for 7 days at 37 °C, TMB measured 0.88, a loss of 6%. The formulation was also maintained for 6 months at a reduced temperature (2-8 °C). A 1:10 dilution on day 0 had an $OD_{285}$ of 0.95. After 6 months the $OD_{285}$ was 0.93, a loss of 2%. When used as an HRP substrate in a conventional reaction mixture the formulation turned from colorless to blue, through green to yellow-orange as described in the literature for TMB.

The formulation was used in a FeLV assay in parallel with a standard two bottle assay method. The assay procedure was as follows. Briefly, microtiter wells were incubated overnight at 2-8°C. with 100 μL of an anti-FeLV antibody to immobilize the antibody to the wells. The solution was removed and wells were air dried and stored covered at 1-8°C. until use. A drop (approximately 40 μL) of sample and a drop of soluble anti-FeLV antibody conjugated to horseradish peroxidase enzyme by standard methods were added each well. After incubation for 5 to 10 min., unbound components were rinsed off by washing each well five times with distilled or deionized water, leaving sample-enzyme conjugate complex bound to the well.

Either two drops of the single bottle substrate or one drop of 0.2 mg/L 3, 3′, 5, 5′-tetramethylbenzidene in acetate/citrate buffer (0.097 M acetate 0.014 M citrate, pH 4.9-5.1) containing 25% dimethylformamide and one drop of 5.8 mM urea hydrogen peroxide in 0.05 potassium phosphate/citrate buffer (pH 4.9-5.1) was added to each well. After 5 to 10 min. incubation, optical density readings of each well were taken at 630 nm.

A lighter color was produced by the single bottle formulation due to a lesser concentration of TMB and higher concentration of solvent than in a standard two bottle assay method but the same results were obtained as illustrated in the table below.

0 279 614

TABLE

| Sample | OD$_{630}$ | |
| --- | --- | --- |
| | Two Bottle Substrate | Single Bottle Substrate |
| Blank | ----- | 0.002 |
| Positive Control | 0.254 | 0.149 |
| Negative Control | 0.001 | 0.001 |
| Standard | 0.181 | 0.085 |
| 33 | 0.000 | 0.002 |
| 34 | 0.003 | 0.001 |
| 46 | 0.711 | 0.282 |
| 50 | 0.001 | 0.001 |
| 51 | 0.000 | 0.000 |
| 55 | 0.001 | 0.001 |
| 56 | 0.008 | 0.006 |
| 57 | 0.002 | 0.003 |
| 58 | 0.006 | 0.005 |
| 70 | 0.001 | 0.002 |
| 71 | 0.004 | 0.003 |
| 73 | 0.005 | 0.004 |

As demonstrated by the table, each of the samples gave the same result as to the presence of FeLV, using either the two bottle or single bottle substrate.

Although 1 drop of each of the components for the two bottle substrate was a larger volume than two drops of the single bottle substrate, a separat experiment demonstrated that increasing the volume did not change the results.

Although the foregoing invention has been described in some detail by way of illustration and example for purposes of clarity of understanding, it will be obvious that certain changes and modifications may be practiced.

**Claims**

1. A storage-stable aqueous substrate solution at a pH in the range of about 5.2 - 6.0, said solution useful for a peroxidase enzyme and comprising in a single container:

(a) an aqueous acetate/citrate buffer having a ratio of acetate to citrate of about 35:1 to about 40:1, and an acetate concentration in said solution of about 0.01 to about 1.0 N;

(b) tetramethylbenzidene at a concentration of at least about 0.04 mM;

(c) a stable source of hydrogen peroxide at a concentration sufficient to produce a color change with tetramethylbenzidine as a result of a peroxidase catalyzed reaction; and

(d) methanol in an amount of about 10% to about 50% v/v.

2. The substrate solution of Claim 1 wherein said acetate concentration is from 0.1 to 0.2 N, and said pH is in the range of 5.4 to 5.8.

3. The substrate solution of Claim 1 or Claim 2 wherein the total buffer molarity is about 0.1 M.

4. The substrate solution of Claim 1, Claim 2 or Claim 3 wherein said acetate concentration is about 0.1 N, citrate concentration is about 0.002 N and said pH is about 5.6.

5. The substrate solution of Claim 1 wherein said tetramethylbenzidine concentration is about 0.2 to

4

about 0.8 mM.

6. The substrate solution of Claim 1 or Claim 5 wherein said tetramethylbenzidine concentration is anout 0.4 mM.

7. The substrate solution of Claim 1 wherein methanol is present at about 30% to about 50% v/v.

8. The substrate of Claim 1 or Claim 7 wherein methanol is present at about 40% v/v.

9. The substrate solution of Claim 1 wherein said stable source of peroxide is urea hydrogen perioxide.

10. The substrate solution of Claim 9 wherein urea hydrogen peroxide is present at a concentration of about 0.002 to 0.005 M.

11. The substrate solution of Claim 1 additionally comprising a bacterial growth inhibitor in an effective amount.

12. The substrate of Claim 11 wherein said bacterial growth inhibitor is sodium benzoate.

13. A method for determining the concentration of a peroxidase in a medium, said method comprising:

combining a medium having horseradish peroxidase with an aqueous subtrate solution at a pH in the range of about 5.2 - 6.0 for a peroxidase enzyme comprising in a single container:

(a) an acetate/citrate buffer having a ratio of acetate to citrate of about 35:1 to about 40:1, an acetate concentration of about 0.1 to about 1.0 N;

(b) tetramethylbenzidine in a concentration of at least about 0.04 mM;

(c) a stable source of hydrogen peroxide at a concentration sufficient to produce a color change with tetramethylbenzidine as a result of a peroxidase catalyst reaction; and

(d) methanol in a concentration of about 10% to about 50% v/v; and

detecting the change in color as as result of the reaction of tetramethylbenzidine.

14. A kit for carrying out a diagnostic assay comprising:

a conjugate of horseradish peroxidase with a ligand or receptor; and an aqueous subtrate solution at a pH in the range of about 5.2 - 6-0 for a peroxidase enzyme comprising in a single container:

(a) an acetate/citrate buffer having a ratio of acetate to citrate of about 35:1 to about 40:1, an acetate concentration of about 0.1 to about 1.0 N;

(b) tetramethylbenzidine in a concentration of at least about 0.04 mM;

(c) a stable source of hydrogen peroxide at a concentration sufficient to produce a color change with tetramethylbenzidine as a result of a peroxidase castalyst reaction; and

(d) methanol in a concentration of about 10% to about 50% v/v.